(19) Europäisches Patentamt
European Patent Office
Office européen des brevets

(11) Veröffentlichungsnummer: **0 373 493 B1**

# (12) EUROPÄISCHE PATENTSCHRIFT

(45) Veröffentlichungstag der Patentschrift: **04.08.93**

(51) Int. Cl.[5]: **C08F 8/44**, C08F 8/14

(21) Anmeldenummer: **89122501.3**

(22) Anmeldetag: **06.12.89**

## (54) Polyacrylsäureester mit quaternären Ammoniumgruppen.

(30) Priorität: **15.12.88 DE 3842201**

(43) Veröffentlichungstag der Anmeldung:
**20.06.90 Patentblatt 90/25**

(45) Bekanntmachung des Hinweises auf die Patenterteilung:
**04.08.93 Patentblatt 93/31**

(84) Benannte Vertragsstaaten:
**BE DE ES FR GB IT NL**

(56) Entgegenhaltungen:
**NL-C- 91 808**
**US-A- 2 435 777**
**US-A- 2 915 481**

(73) Patentinhaber: **Th. Goldschmidt AG**
**Goldschmidtstrasse 100**
**W-4300 Essen 1(DE)**

(72) Erfinder: **Fock, Jürgen, Dr.**
**Mörsenbroicher Weg 114**
**W-4000 Düsseldorf 30(DE)**
Erfinder: **Schaefer, Dietmar, Dr.**
**Milchstrasse 40**
**W-4300 Essen 14(DE)**
Erfinder: **Esselborn, Eberhard**
**Pilotystrasse 21**
**W-4300 Essen 1(DE)**

**EP 0 373 493 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft Polyacrylsäureester eines mittleren Molekulargewichtes von etwa 1.000 bis 50.000 mit quaternären Ammoniumgruppen und deren Verwendung in kosmetischen Zubereitungen, insbesondere in Zubereitungen zur Pflege der Haare und als Textilhilfsmittel, insbesondere zur antistatischen Ausrüstung von Textilfasern und flächigen textilen Produkten.

Polyacrylsäureester mit quaternären Ammoniumgruppen sind bekannt und werden als kationische Hilfsmittel, insbesondere zur Flokkulation von Feststoffteilchen in Schmutzwässern (EP-OS 176 757), zur antistatischen Ausrüstung von Textilfasern (DE-OS 22 42 914), zur Herstellung elektrisch leitender Kopierpapiere oder als Haarfestiger (DE-OS 24 23 182) verwendet.

Die bekannten Polyacrylsäureester mit quaternären Ammoniumgruppen werden entsprechend dem Stand der Technik durch Copolymerisation von Acrylsäureestern, insbesondere Acrylsäuremethylestern und Acrylsäureesterderivaten, welche quaternäre Ammoniumgruppen aufweisen, hergestellt.

Als Acrylsäureesterderivate mit quaternären Ammoniumgruppen werden Verbindungen, wie (Meth)-acryloyloxyethyl- oder (Meth)acryloyloxypropyltrialkyl- bzw. -dialkylbenzyl-Ammoniumhalogenide, verwendet, wobei die Alkylreste insbesondere 1 bis 3 Kohlenstoffatome aufweisen. Zusätzlich können weitere Monomere, wie Styrol, Methyl-, Ethyl-, Butyl-, Dodecyl(meth)acrylat, Vinylacetat, Vinylpropionat, N-Vinylpyrrolidon, Acrylamid, Acrylnitril, copolymerisiert werden.

Bei der radikalischen Copolymerisation der vorgenannten Monomeren entstehen Polymere mit einer sehr weiten Molekulargewichtsverteilung. Die Molekulargewichtsverteilungskurve verläuft somit relativ flach und weist überdies zwei oder mehr Maxima auf, die darauf hindeuten, daß das Polymerisationsprodukt verhältnismäßig heterogen ist. Man kann annehmen, daß der Grund hierfür in den erheblich vom Quotienten 1 abweichenden Copolymerisationsparametern der einzelnen Monomeren zu finden und insbesondere durch den ionischen Charakter der Monomeren mit quaternären Ammoniumgruppen bedingt ist.

Dabei hat sich gezeigt, daß in den durch Copolymerisation erhaltenen Polymerisaten Anteile enthalten sind, die physiologisch bedenklich sind und auch toxische Eigenschaften haben können. Es ist dabei zu vermuten, daß diese unerwünschten Eigenschaften den niedermolekularen Anteilen des Polymerisates zuzuordnen sind. Eine Abtrennung dieser Anteile aus dem Polymerisat ist in wirtschaftlicher Weise nicht möglich. Für viele Einsatzzwecke, insbesondere in der Kosmetik, ist aber die physiologische Unbedenklichkeit von Produkten zwingende Voraussetzung für ihre Verwendbarkeit. Es ist deshalb von besonderem Interesse, Polyacrylsäureester mit quaternären Ammoniumgruppen herzustellen, wobei die Polymerisate frei von physiologisch bedenklichen Bestandteilen sind.

Die Anwendung eines anionischen Polymerisationsverfahrens, wie es in der DE-OS 22 62 588 beschrieben ist, zur Herstellung von Acrylpolymeren mit einem Molekulargewicht von 500 bis 5000 stellt keine brauchbare Lösung der vorgenannten Aufgabe dar. Die Gefahr der Vergelung der Reaktionsprodukte ist hoch. Darüber hinaus ist die Überführung eines anionischen Polymerisationsverfahrens in den Betriebsmaßstab auch aus sicherheitstechnischen Gründen recht problematisch.

Aus der US-A-2 915 481 ist es bekannt, Polymethyl(meth)acrylatharze in Form eines Granulates oder Pulvers mit Mono- oder Dialkanolaminen zu erhitzen, um durch eine Umesterungsreaktion auf der Oberfläche der Harzpartikel primäre oder sekundäre Aminogruppen zu erzeugen, die es gestatten, die Harzgranulate oder Harzpulver zur Entfernung saurer Materialien, wie Mercaptane oder Hydrogensulfide, aus Gasgemischen, Erdölfraktionen oder dergleichen, zu verwenden. Es handelt sich bei den Verfahrensprodukten somit um reversibel beladbare Austauscherharze.

Der Erfindung liegt die Aufgabe zugrunde, Polyacrylsäureester eines mittleren Molekulargewichtes von etwa 1.000 bis 50.000 mit quaternären Ammoniumgruppen herzustellen, wobei die Polymerisate möglichst geringe Anteile an niedermolekularen Produkten enthalten und physiologisch unbedenklich sein sollen.

Es wurde gefunden, daß Polymerisate, welche nur geringe Anteile an niedermolekularen Produkten enthalten, durch Umesterung geeigneter Polyacrylsäureester mit einem Gemisch ausgewählter Aminoalkohole und langkettiger aliphatischer Alkohole erhalten werden können. Dabei werden als Zwischenprodukte Acrylsäureester mit tertiären Aminogruppen gebildet, die durch Quaternierung in die gewünschten Polyacrylsäureester mit quaternären Ammoniumgruppen überführt werden können.

Gegenstand der Erfindung sind deshalb Polyacrylsäureester eines mittleren Molekulargewichtes von etwa 1.000 bis 50.000 mit quaternären Ammoniumgruppen, erhältlich durch Umesterung von durch radikalische Polymerisation erhaltenen Polyacrylsäurealkylestern, deren Alkylreste 1 bis 4 Kohlenstoffatome aufweisen, mit

a) Verbindungen der allgemeinen Formel

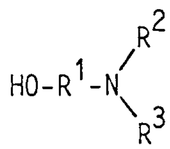

I

$R^1$ = zweiwertiger Rest der allgemeinen Formel

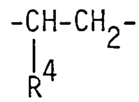

oder $-(C_nH_{2n}O)_m-C_pH_{2p}-$

$R^4$ = Wasserstoff- oder Alkylrest mit 1 bis 16 Kohlenstoffatomen,

n = 2, 3 oder 4,

m = 1 bis 20,

p = 2, 3 oder 4,

$R^2$, $R^3$ = jeweils Alkylrest mit 1 bis 18 Kohlenstoffatomen, und

b) gesättigten oder ungesättigten aliphatischen Alkoholen mit 8 bis 22 Kohlenstoffatomen,

wobei das molare Verhältnis der Dialkylaminoalkohole und der Alkanole mit 8 bis 22 Kohlenstoffatomen 1 : 9 bis 9 : 1 beträgt,

in solchen Mengen, daß bis zu 70 % der Estergruppen umgeestert werden, in Gegenwart eines an sich bekannten Umesterungskatalysators, bei Temperaturen von 70 bis 140° C, gegebenenfalls in Gegenwart eines Lösungsmittels, wobei als Zwischenprodukt ein gemischter Polyacrylsäureester mit tertiären Aminogruppen gebildet wird, und anschließende Quaternierung des Zwischenproduktes mit Alkyl- oder Alkylarylhalogeniden der allgemeinen Formel

$R^5X$

$R^5$ = Alkylrest mit 1 bis 4 Kohlenstoffatomen oder Benzylrest,

X = Halogenatom,

oder mit Dimethyl- oder Diethylsulfat in an sich bekannter Weise bei Temperaturen von 20 bis 140° C und gegebenenfalls erhöhtem Druck.

Im Aminoalkohol der Formel I ist $R^1$ ein zweiwertiger Rest der Formel

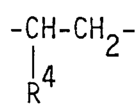

oder $-(C_nH_{2n}O)_m-C_pH_{2p}-$

Beispiele solcher Reste sind $-CH_2-CH_2-$;

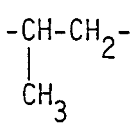 ; 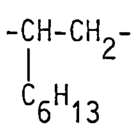 ;

$-(CH_2)_2O(CH_2)_2-$;

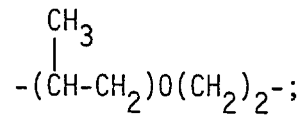

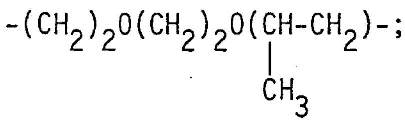
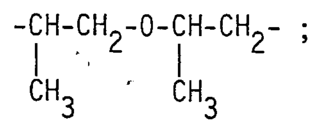

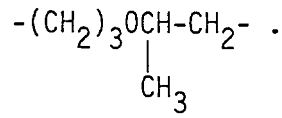

Besonders bevorzugt sind Reste, bei denen $R^4$ ein Wasserstoff-, Methyl- oder Ethylrest ist. Bei den Etherresten sind solche bevorzugt, bei denen n und p einen Wert von 2 oder 3 haben. m hat vorzugsweise einen Wert von 1 bis 10.

$R^2$ und $R^3$ sind jeweils Alkylreste mit 1 bis 18 Kohlenstoffatomen. Die Alkylreste können geradkettig oder verzweigt sein. Bevorzugt sind Alkylreste mit 1 bis 6 Kohlenstoffatomen. Insbesondere bevorzugt sind Alkylreste mit 1 bis 4 Kohlenstoffatomen, wie der Methyl-, Ethyl-, Propyl-, iso-Propyl-, Butyl- und iso-Butylrest.

Innerhalb des polymeren Moleküls können die Reste und Indices verschiedene Bedeutung bzw. Werte annehmen. Hierdurch bedingt können im durchschnittlichen Polymerenmolekül die Indices auch gebrochene Zahlenwerte annehmen.

Der gesättigte oder ungesättigte aliphatische Alkohol b) hat 8 bis 22 Kohlenstoffatome. Besonders bevorzugt sind Alkohole mit 10 bis 18 Kohlenstoffatomen, wobei auch Gemische von Alkoholen verschiedener Kettenlänge verwendet werden können. Beispiele geeigneter Alkohole sind der Octyl-, Decyl-, Dodecyl-, Hexadecyl-, Octadecylalkohol, Behenylalkohol, Oleylalkohol oder die synthetischen Alkohole mit der gewünschten Kohlenstoffanzahl.

Das molare Verhältnis der Dialkylaminoalkanole a) zu den gesättigten oder ungesättigten aliphatischen Alkoholen b) beträgt 1 : 9 bis 9 : 1, wobei ein Bereich von 1 : 3 bis 3 : 1 bevorzugt ist.

Für die Umesterung werden Polyacrylsäurealkylester, deren Alkylreste 1 bis 4 Kohlenstoffatome aufweisen, eingesetzt. Besonders bevorzugt werden deshalb die Methyl-, Ethyl- oder Butylester der Polyacrylsäure für die Umesterung ausgewählt.

Die Umesterung der Polyacrylsäureester mit dem Gemisch aus Dialkylaminoalkoholen und langkettigen aliphatischen Alkoholen erfolgt in an sich bekannter Weise. Sie läuft zweckmäßig in Gegenwart eines an sich bekannten Umesterungskatalysators, wie z. B. Alkyltitanaten oder Alkalialkoholaten, ab und wird gegebenenfalls in Gegenwart eines Lösungsmittels, wie z. B. Toluol, Xylol oder Benzinfraktionen eines Siedebereiches von 80 bis 160° C durchgeführt. Das Lösungsmittel dient in erster Linie dazu, den bei der Umesterung freigesetzten Alkohol aus dem Reaktionsgemisch auszutragen. Dabei begrenzt das verwendete Lösungsmittel die Umesterungstemperaturen, die in einem Bereich von 70 bis 140° C liegen sollen.

Die Umesterungsreaktion soll dabei in solchen Mengenverhältnissen erfolgen, daß bis zu 70 %, vorzugsweise 5 bis 50 %, insbesondere bevorzugt 20 bis 50 %, der Alkylester umgeestert werden.

Als Zwischenprodukt wird dabei der Polyacrylsäureester mit tertiären Aminogruppen erhalten. Diese Verbindungen können ebenfalls im Bereich der Textilhilfsmittel und im kosmetischen Bereich, insbesondere für die Haarkosmetik eingesetzt werden.

In einem zweiten Verfahrensschritt wird das erhaltene Zwischenprodukt in ebenfalls bekannter Weise mit Alkyl- oder Alkylarylhalogeniden oder Dimethyl- bzw. Diethylethersulfat quaterniert.

Als Alkyl- oder Alkylarylhalogenide verwendet man solche der allgemeinen Formel $R^5X$, wobei $R^5$ ein Alkylrest mit 1 bis 4 Kohlenstoffatomen oder ein Benzylrest und X ein Halogenrest ist. Beispiele solcher

Halogenverbindungen sind Methylchlorid, Ethylchlorid, Butylchlorid, Methylbromid oder Ethylbromid.

Die Quaternierung erfolgt bei Temperaturen von 20 bis 140° C und in Abhängigkeit vom Siedepunkt des eingesetzten Quaternierungsmittels, gegebenenfalls in einem geschlossenen System bei erhöhtem Druck.

Innerhalb der erfindungsgemäßen Polyacrylsäureester mit quaternären Ammoniumgruppen sind diejenigen besonders bevorzugt, die durch Umesterung von Polyacrylsäuremethylestern mit einem Gemisch aus Dimethyl- oder Diethylaminoethanol und aliphatischen Alkohlen einer Kettenlänge von 8 bis 20, vorzugsweise 10 bis 18 Kohlenstoffatomen, wobei 20 bis 50 % der Estergruppen umgeestert werden, und anschließende Quaternierung mit Methylchlorid erhältlich sind.

Es ist dem Fachmann klar, daß man anstelle der Homopolymerisate auch Copolymerisate von Acrylsäureestern mit anderen polymerisierbaren Monomeren für die Umesterungsreaktion einsetzen kann. Beispiele solcher Comonomeren sind Styrol, Methyl-, Ethyl-, Butylmethacrylat, Acrylamid, Acrylnitril.

In einer besonderen Ausgestaltungsform der Erfindung sind bis zu 50 Mol-% der Acrylsäureester durch die entsprechenden Methacrylsäureester ersetzt.

Im Gegensatz zu den nach dem Stand der Technik durch Copolymerisation von Acrylsäureestern oder Acrylsäureesterderivaten mit quaternären Ammoniumgruppen erhaltenen Copolymerisaten sind die erfindungsgemäßen Polymerisate polymer einheitlicher aufgebaut. Der Gehalt an niedermolekularen Anteilen ist ganz wesentlich vermindert. Die erfindungsgemäßen Copolymerisate haben keine toxischen oder sonstige physiologisch bedenklichen Eigenschaften.

Ein weiterer Gegenstand der Erfindung besteht in deren Verwendung in kosmetischen Zubereitungen, welche erst durch die gute Verträglichkeit der Verbindungen möglich geworden ist. Die Polymerisate eignen sich insbesondere als Zusatzstoffe für Zubereitungen zur Pflege der Haare. Sie verbessern bereits in geringen Anwendungskonzentrationen von 0,2 bis 2 Gew.-%, bezogen auf Zubereitung, die Kämmbarkeit der Haare, insbesondere nasser Haare, und verringern deren statische Aufladung.

Eine weitere bevorzugte Verwendung der erfindungsgemäßen Verbindungen bietet sich im Textilhilfsmittelbereich an. Sie können dort als Antistatika für die Ausrüstung von Textilfasern und von Textilprodukten, wie Geweben oder Gewirken oder Vliesen, und zur Verbesserung des Griffs verwendet werden. Da derartig ausgerüstete Produkte mit dem menschlichen Körper in Berührung kommen, ist es auch hier von Vorteil, daß die erfindungsgemäßen Verbindungen physiologisch unbedenklich sind.

## Beispiel 1

Herstellung von Polymethylacrylat durch radikalische Polymerisation (nicht erfindungsgemäß)

Eine Lösung von 0,6 g Azodiisobuttersäurenitril und 20,2 g Dodecylmercaptan in 50 g Toluol und 280 g (ca. 3,25 Mol) Methylacrylat wird innerhalb von 2 h in einen mit 53 g Toluol gefüllten Reaktor gegeben; das vorgelegte Lösungsmittel hat dabei eine Temperatur von 100° C und befindet sich unter einer Stickstoffatmosphäre. Danach werden nochmal 0,9 g Azodiisobuttersäurenitril, gelöst in 20 g Methylethylketon, innerhalb von 0,5 h nachgegeben. Schließlich wird das Reaktionsgemisch noch für 1 h bei der gleichbleibenden Temperatur von 100° C weiter erwärmt. Nach Beendigung der Reaktion wird das Lösungsmittel abdestilliert. Es verbleibt eine farblose, viskose Flüssigkeit mit einem Brechungsindex von 1,4802. Aus der gelchromatographischen Untersuchung ergibt sich für das erhaltene Polymerisat ein numerisches Molekulargewicht $\overline{M}_n$ von 1950 und für das Gewichtsmittel des Molekulargewichtes $\overline{M}_w$ 3330; der Uneinheitlichkeitskoeffizient beträgt demnach 1,71. Der Restmonomerengehalt beträgt 0,1 %.

## Beispiele 2 und 3

Herstellung von Polymethylacrylaten höheren Molekulargewichtes durch radikalische Polymerisation (nicht erfindungsgemäß)

Es wird verfahren wie in Beispiel 1 mit der Ausnahme, daß der Gehalt an Dodecylmercaptan gesenkt wird. In Tabelle 1 wird die Abhängigkeit der Zahlen- und Gewichtsmittel des Mclekulargewichts vom Gehalt an Dodecylmercaptan gezeigt.

Tabelle 1

| Polymethylacrylat aus Beispiel | Dodecylmercaptan [Gew.-%] | Molekulargewicht $\overline{M}_n$ | Molekulargewicht $\overline{M}_w$ | Uneinheitlichkeitskoeffizient |
|---|---|---|---|---|
| 2 | 2,95 | 4 453 | 11 346 | 2,55 |
| 3 | 0,43 | 16 750 | 68 500 | 4,09 |

Beispiel 4 A

Umesterung von Polymethylacrylat mit Diethylaminoethanol und Talgfettalkohol

92,3 g des Polymethylacrylates aus Beispiel 1, gelöst in 93 g Toluol, werden zusammen mit 29,3 g (0,25 Mol) Diethylaminoethanol und 65,0 g (0,25 Mol) Talgfettalkohol unter Stickstoff auf 120° C erhitzt. Zunächst werden möglicherweise vorhandene Spuren von Wasser durch azeotrope Destillation entfernt. Danach erfolgt die Zugabe von 0,4 g Natriummethylat als Umesterungskatalysator. Das bei der Umesterung entstehende Methanol wird durch Fraktionierung vom Toluol getrennt. Nach 2 h und nach 4 h werden jeweils 0,35 g Natriummethylat nachdosiert. Die Reaktion ist nach etwa 6 h beendet; ihr Ende wird durch eine Kopftemperatur von etwa 110° C angezeigt.

Der durch gaschromatographische Untersuchung ermittelte Anteil an Diethylaminoethanol von 0,28 % entspricht einem Umsatz bei der Umesterung von 97,8 %. Im Ansatz befinden sich noch 1,2 % Talgfettalkohol, was einem Umsatz von 95,7 % entspricht; der Methanolgehalt im Destillat beträgt 31,1 %, entsprechend einem Umsatz von 93,9 %.

Beispiel 4 B

Quaternierung des Diethylaminoethyl- und Alkylgruppen enthaltenden Polymethylacrylates

Bei dem aus Beispiel 4 A erhaltenen Produkt wird Toluol als Lösungsmittel durch Isopropanol ersetzt. 200 g des im Gewichtsverhältnis 1 : 1 mit dem Lösungsmittel verdünnten Polymerisates werden unter Stickstoff auf 110° C erhitzt und durch Einleiten von Methylchlorid ein Druck von 3,5 bis 4 bar eingestellt, der während der Reaktion durch Nachdosierung konstant gehalten wird.

Aus der Bestimmung des Chloridgehaltes und der Aminzahl ergibt sich ein Umsatz bei der Quaternierung von 98,5 bzw. 98,7 %.

Beispiele 5 A - 12 A

Umesterung von Polymethylacrylaten unterschiedlichen Molekulargewichtes mit verschiedenen Dialkylaminoalkanolen und Fettalkoholen

Es wird verfahren wie in Beispiel 4 A mit der Ausnahme, daß unterschiedliche Dialkylaminoalkanole und Fettalkohole in, hinsichtlich der Methylestergruppen, wechselnden Molekularverhältnissen verwendet werden. Anstelle von Natriummethylat wird in einigen Fällen Isopropyltitanat als Katalysator verwendet. In Tabelle 2 werden Molekulargewicht und Menge des eingesetzten Polymethylacrylates, Art und Menge des Dialkylaminoethanols, Art und Menge des Fettalkohols, Art und Menge des Umesterungskatalysators, der theoretische Substitutionsgrad, der sich aus dem Quotienten der Zahl der substituierten Estergruppen und der Zahl der ursprünglich vorhandenen Estergruppen ergibt, und die Reaktionsausbeuten aus der freigesetzten Menge an Methanol und aus der Aminzahl angegeben.

Tabelle 2

| Beispiel Nr. | Polymethyl-acrylat [ $\bar{M}_n$ ]Menge [g] | Dialkylamino-alkanol Art/Menge [g] | Fettalkohol Art/Menge [g] | Katalysator Art/Menge [g] | Substitutionsgrad der Theorie* [ % ] | Umsatz aus Methanolmenge [ % ] | Umsatz aus Aminzahl [ % ] |
|---|---|---|---|---|---|---|---|
| 5 A | 1950/92,3 | DEAE/29,3 | OCA/66,3 | NAM/1,1 | 50 | 98,2 | 97,8 |
| 6 A | 1950/92,3 | DEAE/29,3 | DDO/46,5 | NAM/1,1 | 50 | 99,1 | 99,0 |
| 7 A | 1950/92,3 | DMAE/26,7 | TFA/78,0 | IPT/1,8 | 60 | 99,3 | 98,5 |
| 8 A | 1950/92,3 | DMAE/35,6 | OCA/39,8 | IPT/1,6 | 55 | 97,6 | 98,2 |
| 9 A | 1950/92,3 | DEAE/29,3 | STA/54,1 | IPT/1,6 | 45 | 98,4 | 96,8 |
| 10 A | 4453/88,7 | DEAE/46,9 | DDO/37,2 | IPT/1,4 | 60 | 99,5 | 99,1 |
| 11 A | 4453/88,7 | DEAE/23,4 | DDO/55,8 | IPT/1,5 | 50 | 97,4 | 98,4 |
| 12 A | 16750/86,5 | DEAE/46,9 | TFA/39,0 | IPT/1,7 | 55 | 98,7 | 98,0 |

$$* \text{ Substitutionsgrad der Theorie} = \frac{\text{Zahl der substituierten Estergruppen}}{\text{Zahl der ursprünglichen vorhandenen Estergruppen}}$$

Legende:

| | | |
|---|---|---|
| DMAE | = | Dimethylaminoethanol |
| DEAE | = | Diethylaminoethanol |
| DDO | = | Dodecanol-1 |
| STA | = | Stearylalkohol |
| TFA | = | Talgfettalkohol ($C_{16}$-$C_{18}$-Alkohol) |
| OCA | = | Ocenol (Oleyl-Cetyl-Alkohol) |
| NAM | = | Natriummethylat |
| IPT | = | Isopropyltitanat |

Beispiele 5 B bis 12 B

Quaternierung der Dialkylaminoalkyl- und Alkylgruppen enthaltenden Polymethylacrylate

Es wird verfahren wie in Beispiel 4 B, mit der Ausnahme, daß bei der Quaternierung der Umesterungsprodukte 5 A bis 12 A in einigen Fällen Benzylchlorid verwendet wird. In Tabelle 3 werden die Umsätze bei der Quaternierung anhand der Chlorwerte und der Aminzahlen gezeigt.

Tabelle 3

| Beispiel Nr. | Umesterungsprodukt aus Beispiel Nr. | Quaternierungsagens Art/Menge [g] | Lösungsmittel | Umsatz aus Chloridwert [%] | Umsatz aus Aminzahl [%] |
|---|---|---|---|---|---|
| 5 B | 5 A | MC/ 7,32 | IPA | 98,3 | 99,0 |
| 6 B | 6 A | MC/ 8,30 | IPA | 99,1 | 98,5 |
| 7 B | 7 A | BC/21,35 | PD | 98,1 | 97,9 |
| 8 B | 8 A | MC/13,46 | IPA | 97,8 | 98,6 |
| 9 B | 9 A | MC/ 7,83 | IPA | 98,7 | 99,2 |
| 10 B | 10 A | MC/12,63 | PD | 97,3 | 98,5 |
| 11 B | 11 A | BC/16,67 | IPA | 96,8 | 97,8 |
| 12 B | 12 A | MC/13,04 | IPA | 97,8 | 98,7 |

Legende:
MC = Methylchlorid
BC = Benzylchlorid
IPA = Isopropylalkohol
PD = 1,2-Propandiol

Beispiel 13

Herstellung eines quaternäre Ammonium- und langkettige Alkylgruppen enthaltenden Copolymerisates durch radikalische Polymerisation (nicht erfindungsgemäß)

Eine Lösung von 0,6 g Azodiisobuttersäurenitril und 20,2 g Dodecylmercaptan in 72,2 g (ca. 0,84 Mol) Methylacrylat, 71,8 g (ca. 0,42 Mol) Diethylaminoethylacrylat und 135,8 g (0,42 Mol) Stearylacrylat, gelöst in 50 g Toluol, werden innerhalb von 2 h in einen mit 53 g Toluol gefüllten Reaktor gegeben; das vorgelegte Lösungsmittel hat dabei eine Temperatur von 100 °C und befindet sich unter einer Stickstoffatmosphäre. Danach werden nochmals 0,9 g Azodiisobuttersäurenitril in 20 g Methylethylketon innerhalb von 0,5 h nachgegeben und schließlich das Reaktionsgemisch für 1 h bei der gleichbleibenden Temperatur von 100 °C weiter erwärmt. Nach Beendigung der Reaktion werden Lösungsmittel und nicht umgesetzte Monomere durch Destillation im Vakuum entfernt.

Aus der gelchromatographischen Untersuchung ergibt sich für das erhaltene Polymerisat ein numerisches Molekulargewicht $\overline{M}_n$ von 1370 und für das Gewichtsmittel des Molekulargewichtes $\overline{M}_w$ 3150; der Uneinheitlichkeitskoeffizient beträgt demnach 2,3. Der Restmonomerengehalt wird zu 0,1 % ermittelt.

Das erhaltene Produkt wird zunächst mit 300 g Isopropanol verdünnt und in einem Druckreaktor auf 110 °C unter Stickstoff erwärmt. Dann wird soviel Methylchlorid eingeleitet, daß sich ein Druck von 3,5 bis 4 bar einstellt; dieser wird während der Reaktion durch Nachdosierung konstant gehalten. Die Reaktionsdauer beträgt 5 h.

Aus der Bestimmung des Chloridgehaltes und der Aminzahl ergibt sich ein Umsatz bei der Quaternierung von 97,9 bzw. 99,0 %.

Im Gelphasenchromatogramm zeigt sich im niedermolekularen Bereich ein Peak, der auf die Gegenwart niedermolekularer Anteile hinweist.

Anwendungstechnische Beispiele

Herstellung und Überprüfung von Haarbehandlungsmitteln unter Verwendung der in den Beispielen 4 B bzw. 12 B hergestellten Umesterungsprodukte mit Quat-Gruppen.

Konditioniershampoo:

| | | |
|---|---|---|
| A | Tego®-Betain L7 1): | 2 Gew.-% |
| | Antil® 141 Liquid 2): | 3 Gew.-% |
| B | Umesterungspdrodukt aus Beispiel 4 B: | 0,5 Gew.-% |
| C | Natriumlaurylethersulfat: | 10 Gew.-% |
| D | Wasser: | 84,5 Gew.-% |

Zur Herstellung des Haarbehandlungsmittels werden die Bestandteile in der aufgeführten Reihenfolge (A - D) zusammengegeben. Jede Mischung muß vor der Zugabe weiterer Komponenten klar gelöst sein.

1) Tego®-Betain L7 = Cocamidopropyl-Betain (1-Alkoylamino-3-dimethyl-ammonium-propan-3-carboxymethyl-betain)

2) Antil® 141 Liquid ist ein flüssiges Verdickungsmittel auf der Basis eines nichtionogenen Fettsäurepolyalkylenglykolesters.

Cremespülung:

| | | |
|---|---|---|
| A | Teginacid® X 3): | 6,0 Gew.-% |
| | Cetylalkohol: | 0,5 Gew.-% |
| B | Umesterungsprodukt aus Beispiel 12 B: | 1,0 Gew.-% |
| | Wasser: | 92,5 Gew.-% |

Zur Herstellung der Zubereitung werden A und B bei 70°C zusammengegeben, homogenisiert und unter Rühren abgekühlt.

3) Teginacid® X ist ein O/W-Emulgator auf der Basis einer Mischung von Glycerinmono-/-distearaten mit Polyglykolfettalkoholethern.

In der praktischen Anwendung beim halbseitigen Vergleichstest auf menschlichem Haar ergibt sich gegenüber Shampooformulierungen bzw. Cremespülungen mit polyquaternären Verbindungen aus dem Stand der Technik:

- eine höhere antistatische Wirkung
- eine verbesserte Naßkämmbarkeit
- eine verbesserte Fülle und Haltbarkeit der Frisur
- eine Verbesserung des Glanzes der Haare

**Patentansprüche**

**Patentansprüche für folgende Vertragsstaaten : BE, DE, FR, GB, IT, NL**

**1.** Polyacrylsäureester eines mittleren Molekulargewichtes von etwa 1.000 bis 50.000 mit quaternären Ammoniumgruppen, erhältlich durch Umesterung von durch radikalische Polymerisation erhaltenen Polyacrylsäurealkylestern, deren Alkylreste 1 bis 4 Kohlenstoffatome aufweisen, mit

a) Verbindungen der allgemeinen Formel

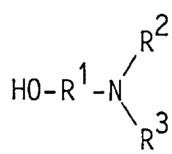

$R^1$ = zweiwertiger Rest der allgemeinen Formel

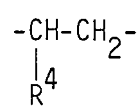

oder $-(C_nH_{2n}O)_m-C_pH_{2p}-$

$R^4$ = Wasserstoff- oder Alkylrest mit 1 bis 16 Kohlenstoffatomen,

n = 2, 3 oder 4,

m = 1 bis 20,

p = 2, 3 oder 4,

$R^2$, $R^3$ = jeweils Alkylrest mit 1 bis 18 Kohlenstoffatomen, und

b) gesättigten oder ungesättigten aliphatischen Alkoholen mit 8 bis 22 Kohlenstoffatomen,

wobei das molare Verhältnis der Dialkylaminoalkohole und der Alkanole mit 8 bis 22 Kohlenstoffatomen 1 : 9 bis 9 : 1 beträgt,

in solchen Mengen, daß bis zu 70 % der Estergruppen umgeestert werden, in Gegenwart eines an sich bekannten Umesterungskatalysators, bei Temperaturen von 70 bis 140° C, gegebenenfalls in Gegenwart eines Lösungsmittels, wobei als Zwischenprodukt ein gemischter Polyacrylsäureester mit tertiären Aminogruppen gebildet wird, und anschließende Quaternierung des Zwischenproduktes mit Alkyl- oder Alkylarylhalogeniden der allgemeinen Formel

$R^5X$

$R^5$ = Alkylrest mit 1 bis 4 Kohlenstoffatomen oder Benzylrest,
X = Halogenatom,
oder mit Dimethyl- oder Diethylsulfat in an sich bekannter Weise bei Temperaturen von 20 bis 140° C und gegebenenfalls erhöhtem Druck.

**2.** Polyacrylsäureester mit tertiären Aminogruppen, erhältlich als Zwischenprodukt gemäß Anspruch 1.

**3.** Polyacrylsäureester nach Anspruch 1, erhältlich durch Umesterung von Polyacrylsäuremethylestern mit einem Gemisch von a) Dimethyl- oder Diethylaminoethanol und b) aliphatischen Alkoholen einer Kettenlänge von 8 bis 20 Kohlenstoffatomen in solchen Mengen, daß 20 bis 50 % der Estergruppen umgeestert werden, und anschließende Quaternierung mit Methylchlorid.

**4.** Polyacrylsäureester nach Anspruch 1 oder 3, erhältlich durch Umesterung von Polyacrylsäuremethylestern mit einem Gemisch von a) Dialkylaminoalkanolen und b) aliphatischen Alkoholen, wobei das molare Verhältnis der Alkohole a) : b) gleich 1 : 3 bis 3 : 1 ist.

**5.** Polyacrylsäureester nach Anspruch 1, dadurch gekennzeichnet, daß bis zu 50 Mol-% der Acrylsäureester durch die entsprechenden Methacrylsäureester ersetzt sind.

**6.** Verwendung der quaternäre Ammoniumgruppen aufweisenden Polyacrylsäureester nach einem oder mehreren der Ansprüche 1, 3, 4 oder 5 in kosmetischen Zubereitungen, insbesondere zur Pflege der Haare.

**7.** Verwendung der quaternäre Ammoniumgruppen aufweisenden Polyacrylsäureester nach einem oder mehreren der Ansprüche 1, 3, 4 oder 5 als Antistatika für die Ausrüstung von Textilfasern oder textilen Produkten.

**Patentansprüche für folgenden Vertragsstaat : ES**

**1.** Verfahren zur Herstellung von Polyacrylsäureestern eines mittleren Molekulargewichtes von etwa 1.000 bis 50.000 mit quaternären Ammoniumgruppen, dadurch gekennzeichnet, daß man durch radikalische Polymerisation erhaltene Polyacrylsäureester, deren Alkylreste 1 bis 4 Kohlenstoffatome aufweisen, wobei bis zu 50 Mol-% der Acrylsäureester durch die entsprechenden Methacrylsäureester ersetzt sein können, zunächst mit

a) Verbindungen der allgemeinen Formel

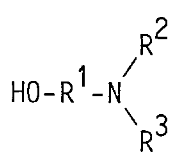

$R^1$ = zweiwertiger Rest der allgemeinen Formel

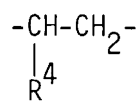

oder $-(C_nH_{2n}O)_m-C_pH_{2p}-$
$R^4$ = Wasserstoff- oder Alkylrest mit 1 bis 16 Kohlenstoffatomen,
n = 2, 3 oder 4,
m = 1 bis 20,
p = 2, 3 oder 4,
$R^2$, $R^3$ = jeweils Alkylrest mit 1 bis 18 Kohlenstoffatomen, und

b) gesättigten oder ungesättigten aliphatischen Alkoholen mit 8 bis 22 Kohlenstoffatomen,
wobei das molare Verhältnis der Dialkylaminoalkohole und der Alkanole mit 8 bis 22 Kohlenstoffatomen 1 : 9 bis 9 : 1 beträgt, in solchen Mengenverhältnissen umestert, daß bis zu 70 % der Estergruppen umgeestert werden, wobei man die Reaktion in Gegenwart eines an sich bekannten Umesterungskatalysators bei Temperaturen von 70 bis 140° C, gegebenenfalls in Gegenwart eines Lösungsmittels durchführt, wobei als Zwischenprodukt ein gemischter Polyacrylsäureester mit tertiären Aminogruppen gebildet wird, und sodann das erhaltene Zwischenprodukt mit Alkyl- oder Alkylarylhalogeniden der allgemeinen Formel

$R^5X$

$R^5$ = Alkylrest mit 1 bis 4 Kohlenstoffatomen oder Benzylrest,
X = Halogenatom,
oder mit Dimethyl- oder Diethylsulfat in an sich bekannter Weise bei Temperaturen von 20 bis 140° C und gegebenenfalls erhöhtem Druck quaterniert.

**2.** Verfahren nach Anspruch 1, dadurch gekennzeichnet, daß man Polyacrylsäuremethylester mit einem Gemisch von a) Dimethyl- oder Diethylaminoethanol und b) aliphatischen Alkoholen einer Kettenlänge von 8 bis 20 Kohlenstoffatomen in solchen Mengen umsetzt, daß 20 bis 50 % der Estergruppen umgeestert werden, und anschließend das erhaltene Zwischenprodukt mit Methylchlorid quaterniert.

**3.** Verfahren nach Anspruch 1 oder 2, dadurch gekennzeichnet, daß man Polyacrylsäuremethylester mit einem Gemisch von a) Dialkylaminoalkanolen und b) aliphatischen Alkoholen im Mengenverhältnis a) : b) gleich 1 : 3 bis 3 : 1 umestert.

**4.** Verwendung der nach einem Verfahren der Ansprüche 1, 2 oder 3 hergestellten Verbindungen in kosmetischen Zubereitungen, insbesondere zur Pflege der Haare.

## Claims

**Claims for the following Contracting States : BE, DE, FR, GB, IT, NL**

**1.** Polyacrylic acid esters of an average molecular weight of about 1,000 to 50,000 and having quaternary ammonium groups, obtainable by transesterification of alkyl polyacrylates obtained by free radical polymerisation, the alkyl radicals of which contain 1 to 4 carbon atoms, with
a) compounds of the general formula

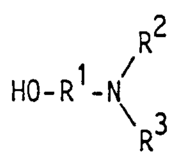

$R^1$ = a divalent radical of the general formula

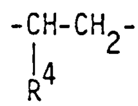

or $-(C_nH_{2n}O)_m-C_pH_{2p}-$
$R^4$ = a hydrogen or alkyl radical having 1 to 16 carbon atoms,
n = 2, 3 or 4,
m = 1 to 20,
p = 2, 3 or 4,
$R^2$ and $R^3$ = in each case an alkyl radical having 1 to 18 carbon atoms, and

b) saturated or unsaturated aliphatic alcohols having 8 to 22 carbon atoms,

where the molar ratio of the dialkylaminoalcohols and the alkanols having 8 to 22 carbon atoms is 1:9 to 9:1,

in amounts such that up to 70% of the ester groups are transesterified, in the presence of a transesterification catalyst known per se, at temperatures from 70 to 140 °C, optionally in the presence of a solvent, a mixed polyacrylic acid ester having tertiary amino groups being formed as an intermediate, and subsequent quaternisation of the intermediate with alkyl or alkylaryl halides of the general formula

$R^5X$

$R^5$ = an alkyl radical having 1 to 4 carbon atoms or a benzyl radical,

X = a halogen atom,

or with dimethyl or diethyl sulphate in a manner known per se at temperatures from 20 to 140 °C and, optionally, at elevated pressure.

**2.** Polyacrylic acid esters having tertiary amino groups, obtainable as intermediates according to Claim 1.

**3.** Polyacrylic acid esters according to Claim 1, obtainable by transesterification of methyl polyacrylates with a mixture of a) dimethyl- or diethylaminoethanol and b) aliphatic alcohols of a chain length of from 8 to 20 carbon atoms in amounts such that 20 to 50% of the ester groups are transesterified, and subsequent quaternisation with methyl chloride.

**4.** Polyacrylic acid esters according to Claim 1 or 3, obtainable by transesterification of methyl polyacrylates with a mixture of a) dialkylaminoalkanols and b) aliphatic alcohols, the molar ratio of the alcohols a):b) being equal to 1:3 to 3:1.

**5.** Polyacrylic acid esters according to Claim 1, characterised in that up to 50 mol% of the acrylic acid esters are replaced by the corresponding methacrylic acid esters.

**6.** Use of the polyacrylic acid esters containing quaternary ammonium groups according to one or more of Claims 1, 3, 4 or 5 in cosmetic compositions, in particular for care of the hair.

**7.** Use of the polyacrylic acid esters containing quaternary ammonium groups according to one or more of Claims 1, 3, 4 or 5 as antistatics for the finishing of textile fibres or textile products.

**Claims for the following Contracting State : ES**

**1.** Process for the preparation of polyacrylic acid esters of an average molecular weight of about 1,000 to 50,000 and having quaternary ammonium groups, characterised in that polyacrylic acid esters obtained by free radical polymerisation, the alkyl radicals of which contain 1 to 4 carbon atoms, it being possible to replace up to 50 mol% of the acrylic acid esters by the corresponding methacrylic acid esters, are transesterified first with

a) compounds of the general formula

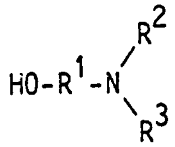

$R^1$ = a divalent radical of the general formula

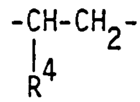

or $-(C_nH_{2n}O)_m-C_pH_{2p}-$

$R^4$ = a hydrogen or alkyl radical having 1 to 16 carbon atoms,
n = 2, 3 or 4,
m = 1 to 20,
p = 2, 3 or 4,
$R^2$ and $R^3$ = in each case an alkyl radical having 1 to 18 carbon atoms, and

b) saturated or unsaturated aliphatic alcohols having 8 to 22 carbon atoms,

where the molar ratio of the dialkylaminoalcohols and the alkanols having 8 to 22 carbon atoms is 1:9 to 9:1, in quantitative ratios such that up to 70% of the ester groups are transesterified, the reaction being carried out in the presence of a transesterification catalyst known per se at temperatures from 70 to 140 °C, optionally in the presence of a solvent, a mixed polyacrylic acid ester having tertiary amino groups being formed as an intermediate, and the intermediate obtained is then quaternised with alkyl or alkylaryl halides of the general formula

$R^5X$

$R^5$ = an alkyl radical having 1 to 4 carbon atoms or a benzyl radical,
X = a halogen atom,

or with dimethyl or diethyl sulphate in a manner known per se at temperatures from 20 to 140 °C and, optionally, at elevated pressure.

2. Process according to Claim 1, characterised in that methyl polyacrylates are reacted with a mixture of a) dimethyl- or diethylaminoethanol and b) aliphatic alcohols of a chain length of from 8 to 20 carbon atoms in amounts such that 20 to 50% of the ester groups are transesterified, and the intermediate obtained is subsequently quaternised with methyl chloride.

3. Process according to Claim 1 or 2, characterised in that methyl polyacrylate is transesterified with a mixture of a) dialkylaminoalkanols and b) aliphatic alcohols in a quantitative ratio a):b) equal to 1:3 to 3:1.

4. Use of the compounds prepared according to a process of Claims 1, 2 or 3 in cosmetic compositions, in particular for care of the hair.

## Revendications

### Revendications pour les Etats contractants suivants : BE, DE, FR, GB, IT, NL

1. Polyacrylates d'un poids moléculaire moyen compris entre environ 1 000 et 50 000, contenant des groupes d'ammonium quaternaire, qu'on obtient grâce à la transestérification de polyacrylates d'alkyle obtenus par polymérisation radicalaire, dont les restes alkyles comportent de 1 à 4 atomes de carbone, avec

a) des composés de formule générale :

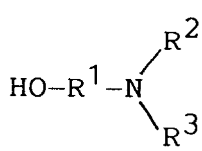

$R^1$ = reste bivalent répondant à la formule générale

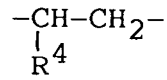

ou $-(C_nH_{2n}O)_m-C_pH_{2p}-$

$R^4$ = reste d'hydrogène ou alkyle contenant de 1 à 16 atomes de carbone,
n = 2, 3 ou 4,
m = 1 à 20,
p = 2, 3 ou 4,
$R^2$, $R^3$ = chacun un reste alkyle contenant de 1 à 18 atomes de carbone, et

b) des alcools aliphatiques saturés ou insaturés, contenant de 8 à 22 atomes de carbone,

le rapport molaire des dialkylamino-alcools et des alcanols contenant de 8 à 22 atomes de carbone valant de 1:9 à 9:1, en des quantités telles que jusqu'à 70 % des groupes esters sont transestérifiés, en présence d'un catalyseur de transestérification connu en soi, à une température comprise entre 70 et 140 °C, éventuellement en présence d un solvant, un polyacrylate mixte contenant des groupes tertiaires amino étant formé, comme produit intermédiaire, et quaternisation subséquente du produit intermédiaire avec des halogénures d'alkyle ou d'alkylaryle, répondant à la formule générale

$R^5X$

$R^5$ = reste alkyle contenant de 1 à 4 atomes de carbone, ou reste benzyle,
X = atome d'halogène,

ou avec du sulfate de diméthyle ou de diéthyle, de manière connue en sol, à une température comprise entre 20 et 140 °C, et éventuellement à une pression augmentée.

**2.** Polyacrylates contenant des groupes tertiaires amino, obtenus comme produit intermédiaire selon la revendication 1.

**3.** Polyacrylates selon la revendication 1, obtenus par transestérification de polyacrylates de méthyle avec un mélange de a) diméthyl- ou diéthylaminoéthanol et b) alcools aliphatiques d'une longueur de chaîne de 8 à 20 atomes de carbone, en des quantités telles que 20 à 50 % des groupes esters sont transestérifiés, et quaternisation subséquente avec du chlorure de méthyle.

**4.** Polyacrylates selon la revendication 1 ou 3, obtenus par transestérification de polyacrylates de méthyle avec un mélange de a) dialkylaminoalcanols et b) alcools aliphatiques, le rapport molaire des alcools a) : b) valant de 1:3 à 3:1.

**5.** Polyacrylates selon la revendication 1, caractérisés en ce qu'on remplace jusqu'à 50 % en moles des esters de l'acide acrylique par les esters correspondants de l'acide méthacrylique.

**6.** Utilisation des polyacrylates contenant des groupes d'ammonium quaternaire selon une ou plusieurs des revendications 1, 3, 4 ou 5, dans des préparations cosmétiques, en particulier pour les soins des cheveux.

**7.** Utilisation des polyacrylates contenant des groupes d'ammonium quaternaire selon une ou plusieurs des revendications 1, 3, 4 ou 5, comme agents antistatiques pour l'apprêt de fibres textiles ou de produits textiles.

**Revendications pour l'Etat contractant suivant : ES**

**1.** Procédé de préparation de polyacrylates d'un poids moléculaire moyen compris entre environ 1 000 et 50 000, contenant des groupes d'ammonium quaternaire, caractérisé en ce qu'on trapsestérifie des polyacrylates, obtenus par polymérisation radicalaire, dont les restes alkyles comportent de 1 à 4 atomes de carbone, jusqu'à 50 % en moles des esters de l'acide acrylique pouvant être remplacés par les esters correspondants de l'acide méthacrylique, d'abord avec

a) des composés de formule générale :

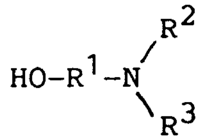

$R^1$ = reste bivalent répondant à la formule générale

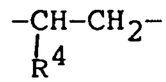

ou $-(C_nH_{2n}O)_m-C_pH_{2p}-$
$R^4$ = reste d'hydrogène ou alkyle contenant de 1 à 16 atomes de carbone,
n = 2, 3 ou 4,
m = 1 à 20,
p = 2, 3 ou 4,
$R^2$, $R^3$ = chacun un reste alkyle contenant de 1 à 18 atomes de carbone, et

b) des alcools aliphatiques saturés ou insaturés, contenant de 8 à 22 atomes de carbone,

le rapport molaire des dialkylamino-alcools et des alcanols contenant de 8 à 22 atomes de carbone valant de 1:9 à 9:1, en des quantités telles que jusqu'à 70 % des groupes esters sont transestérifiés, la réaction étant effectuée en présence d'un catalyseur de transestérification connu en soi, à une température comprise entre 70 et 140 °C, éventuellement en présence d'un solvant, un polyacrylate mixte contenant des groupes tertiaires amino étant formé, comme produit intermédiaire, et ensuite, on quaternise le produit intermédiaire obtenu avec des halogénures d'alkyle ou d'alkylaryle, répondant à la formule générale

$R^5X$

$R^5$ = reste alkyle contenant de 1 à 4 atomes de carbone, ou reste benzyle,
X = atome d'halogène,

ou avec du sulfate de diméthyle ou de diéthyle, de manière connue en soi, à une température comprise entre 20 et 140 °C, et éventuellement à une pression augmentée.

**2.** Procédé selon la revendication 1, caractérisé en ce qu'on fait réagir des polyacrylates de méthyle avec un mélange de a) diméthyl- ou diéthylaminoéthanol et b) alcools aliphatiques d'une longueur de chaîne de 8 à 20 atomes de carbone, en des quantités telles que 20 à 50 % des groupes esters sont transestérifiés et ensuite, on quaternise le produit intermédiaire obtenu avec du chlorure de méthyle.

**3.** Procédé selon la revendication 1 ou 2, caractérisé en ce qu'on transestérifie des polyacrylates de méthyle avec un mélange de a) dialkylaminoalcanols et b) alcools aliphatiques, à un rapport quantitatif a):b) valant de 1:3 à 3:1.

**4.** Utilisation des composés préparés selon un procédé des revendications 1, 2 ou 3, dans des préparations cosmétiques, en particulier pour les soins des cheveux.